# EUROPEAN PATENT APPLICATION

(11) **EP 2 442 558 A1**
(43) Date of publication of application: **18.04.2012**
(21) Application number: 11185072.3
(22) Date of filing: 13.10.2011
(51) Int. Cl.: H04N 5/365, H04N 5/367, A61B 1/00

(54) **Endoscopic device**

(30) Priority: 18.10.2010 JP 2010233354
(71) Applicant: Fujifilm Corporation, Minato-ku Tokyo 106-8620 (JP)
(72) Inventor: Takamatsu, Masaki, Ashigarakami-gun, Kanagawa, 258-8538 (JP)
(74) Representative: Klunker . Schmitt-Nilson . Hirsch

(57) **Abstract**

To provide an endoscopic device (10) in which an endoscope includes a correction means (60) for an image using a correction parameter such as sensitivity unevenness correction or defective pixel correction, the endoscopic device being capable of storing the correction parameter using an existing I/F without further providing a new device or a signal line.

The above-described problem is solved by adopting a configuration in which an endoscopic device (10) includes an endoscope (12) that includes a correction means (60) correcting an image and a storage means (62) storing a correction parameter, a control device (74), and a generating means (70) generating a correction parameter, wherein the correction parameter generated by the generating means in accordance with the command of the control device is written in the storage means (62) by the correction means (60).

## Description

### BACKGROUND OF THE INVENTION

1. Field of the invention

The present invention relates to a technical field of an endoscopic device that acquires an image using a (solid-state) imaging element, and more particularly, to an endoscopic device that corrects an image of an endoscope and easily stores or updates a correction parameter used for correction.

2. Description of the Related Art

An endoscope (an electronic endoscope) has been used so as to diagnose whether a diseased portion is present in a living body or how far the diseased portion has progressed.

The endoscope is operated in a manner such that light is radiated to a part of a living body, the reflected light is photographed by an imaging element such as a CCD sensor, and the acquired image is displayed on a display. Based on the image, a change in the color, the brightness, the structure, or the like of the surface of the living body is observed, which is provided for a doctor to determine the state of the diseased portion.

As is widely known, the imaging element acquiring the image has a configuration in which pixels acquiring the image (points measuring the light amount) are arranged two-dimensionally.

Here, each of the pixels of the imaging element does not have completely uniform characteristics. For example, each of the pixels has a variation in sensitivity (sensitivity unevenness) or the like. Further, the pixels of the imaging element may include a so-called defective pixel which may not output an appropriate signal in accordance with the acquired image (the incident light amount). Furthermore, the balance (so-called white balance) of respective colors of R, G, and B of the image output from the imaging element may not be appropriate.

When the image is acquired in the state where such an imaging element has variations in the characteristics (individual difference), the appropriate image may not be obtained. In particular, in the endoscope used for medical purposes, the diagnosis based on the inappropriate image may cause a critical problem leading to a mistaken diagnosis or the like.

For this reason, in the endoscopic device, the image acquired by the imaging element is subjected to correction such as sensitivity unevenness correction, defective pixel correction, or white balance adjustment, thereby outputting an appropriate image without any degradation in the image quality caused by the individual differences of each pixel.

Further, in the related art, such correction on the image is performed by a processor device that processes an image acquired by an endoscope and displays the result on a display device. However, there are also proposed various devices capable of correcting an image acquired by an endoscope and outputting the result to a processor device.

For example, JP1988-117702A (JP-S63-117702A) discloses an endoscopic device in which an operation means is provided in an endoscope so as to curve a curved portion or suction air/water and the operation means is provided with a circuit correcting sensitivity unevenness, a circuit correcting a defective pixel, and a circuit performing dark current correction (offset correction).

Further, JP2003-153859A discloses an endoscopic device in which a connector provided in an endoscope to be connected to a processor device is provided with a DSP (Digital Signal Processor) performing image correction such as white balance adjustment or a storage means storing a correction parameter used in the image correction of the DSP.

Furthermore, JP2006-212335A discloses an endoscopic device in which a connector of an endoscope is provided with a signal processing circuit converting an image from the format of RGB into the format of YCC and correcting tone or brightness during the conversion or further performing gamma correction or white balance adjustment or is provided with a storage means storing a correction parameter used for image correction in the signal processing circuit.

### SUMMARY OF THE INVENTION

As disclosed in the related art, as an example, sensitivity unevenness correction or white balance adjustment is performed in a manner such that a parameter for correcting a pixel (a correction parameter) is calculated and stored for each pixel in advance and image correction (image processing) is performed for each pixel of the acquired image using the correction parameter.

Further, defective pixel correction is performed in such a manner that a defective pixel is detected and stored in advance and each defective pixel of the acquired image is compensated for by using image data of peripheral pixels.

Accordingly, in the endoscope, when such correction is performed, the correction parameter needs to be stored (written) in a memory included in the endoscope.

Further, individual differences of an imaging element such as a CCD sensor used in the endoscope, such as sensitivity unevenness or defective pixels may change over time. Even when such a temporal change occurs, in order for the endoscope to stably acquire an appropriate image, it is desirable to perform re-calculation of a correction parameter or re-detection of defective pixels, that is, an update of the correction parameter, which is a so-called calibration of the endoscope.

In the endoscopic device disclosed in JP1988-117702A (JP-S63-117702A) or JP2006-212335A, storage or updating of the correction parameter is not considered at all.

On the other hand, in the endoscopic device disclosed in JP2003-153859A, the connector of the endoscope provided with the DSP used for image correction is also provided with a means that corrects a correction parameter for white balance adjustment. However, in the configuration disclosed in JP2003-153859A, there is a need to provide a CCD sensor, a light source, an image analysis means, or the like in the connector in addition to the DSP or the memory used for image correction in order to correct the correction parameter. For this reason, the connector increases in size and increases cost of the endoscope.

An object of the present invention is to solve the problems of the related art and provide an endoscopic device that acquires an image using an imaging element for the purpose of diagnosis, the endoscopic device being capable of outputting an image subjected to image correction using a correction parameter such as sensitivity unevenness correction or defective pixel correction from an endoscope, easily storing a correction parameter in the endoscope without further providing a new device, a signal line, a connector, or the like in addition to a device or a memory used for image correction, and easily updating a correction parameter if necessary when a temporal change occurs in the endoscope.

In order to attain the above-described object, there is provided an endoscopic device including: an endoscope that acquires an image using an imaging element; a control device that is connected to the endoscope; and a parameter generating means that generates a correction parameter for correcting the image acquired by the imaging element, wherein the endoscope includes a correction means that corrects the image acquired by the imaging element using the correction parameter and a storage means that stores the correction parameter, and wherein the parameter generating means generates the correction parameter in accordance with a command generated by the control device, and stores the generated correction parameter in the storage means using the correction means.

In the endoscopic device of the present invention, the control device may include the parameter generating means. At this time, an image signal line used to output the image corrected by the correction means from the endoscope to an external device may be connected to the control device. The correction parameter may be supplied from the control device to the correction means using the image signal line, and the correction means may store the correction parameter in the storage means.

Alternatively, the correction means may include the parameter generating means.

Further, the correction means may be configured as a logic device which is able to rewrite a program therein.

According to the endoscopic device of the present invention with the above-described configuration, the correction parameter may be easily stored (written) in the storage means of the endoscope without further providing a device, a signal line, a connector, or the like other than a correction means correcting an image and a storage means storing a correction parameter, the endoscope may perform image correction using the correction parameter such as sensitivity unevenness correction or defective pixel correction and may easily update the correction parameter even when a temporal change or the like occurs in the endoscope.

For this reason, according to the present invention, it is possible to reliably output an image subjected to image correction using an appropriate correction parameter from the endoscope without causing an increase in size or cost of the endoscope.

### BRIEF DESCRIPTION OF THE DRAWINGS

Fig. 1 is a diagram schematically illustrating an example of an endoscopic device of the present invention.

Fig. 2A is a block diagram schematically illustrating a configuration of a scope portion of an endoscope, and Fig. 2B is a block diagram schematically illustrating a configuration of a video connector.

Fig. 3 is a block diagram schematically illustrating a configuration of the endoscopic device of Fig. 1.

Figs. 4A to 4D are schematic diagrams illustrating an operation of the endoscopic device of the present invention.

### DESCRIPTION OF THE PREFERRED EMBODIMENTS

Hereinafter, an endoscopic device of the present invention will be specifically described with reference to an exemplary embodiment shown in the accompanying drawings.

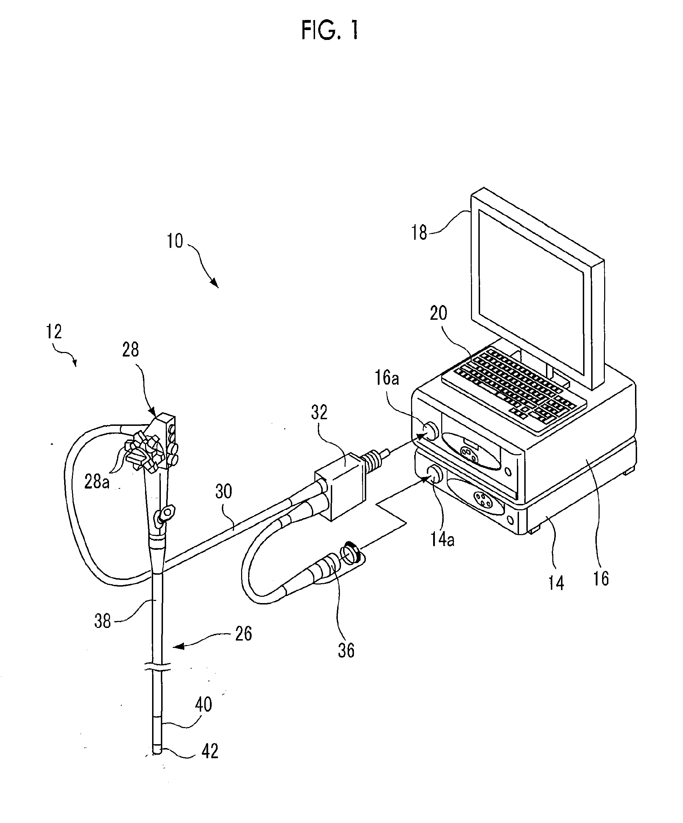

Fig. 1 schematically illustrates an example of an endoscopic device of the present invention.

As an example, an endoscopic device 10 shown in Fig. 1 includes an endoscope 12, a processor device 14 that processes an image acquired by the endoscope 12, a light source device 16 that supplies illumination light used for acquisition (observation) in the endoscope, a display device 18 that displays the image acquired by the endoscope thereon, and an input device 20 which is used to input various commands therethrough.

As an example, in the endoscopic device 10 shown in the drawing, the processor device 14 serves as a control device of the present invention, and includes a parameter generating means.

As shown in Fig. 1, the endoscope 12 includes an insertion means 26, an operation means 28, a universal cord 30, a connector 32, and a video connector 36 as in an ordinary endoscope. Further, as in the ordinary endoscope, the insertion means 26 includes an elongated flexible portion 38 which is provided on the side of the base end, a scope portion (an endoscope front end portion) 42 which is provided on the side of the front end thereof so as to dispose a CCD sensor 48 and the like therein, and a curved portion (an angle portion) 40 which is provided between the flexible portion 38 and the scope portion 42. Furthermore, the operation means 28 is provided with an operation knob 28a or the like which is used to curve the curved portion 40.

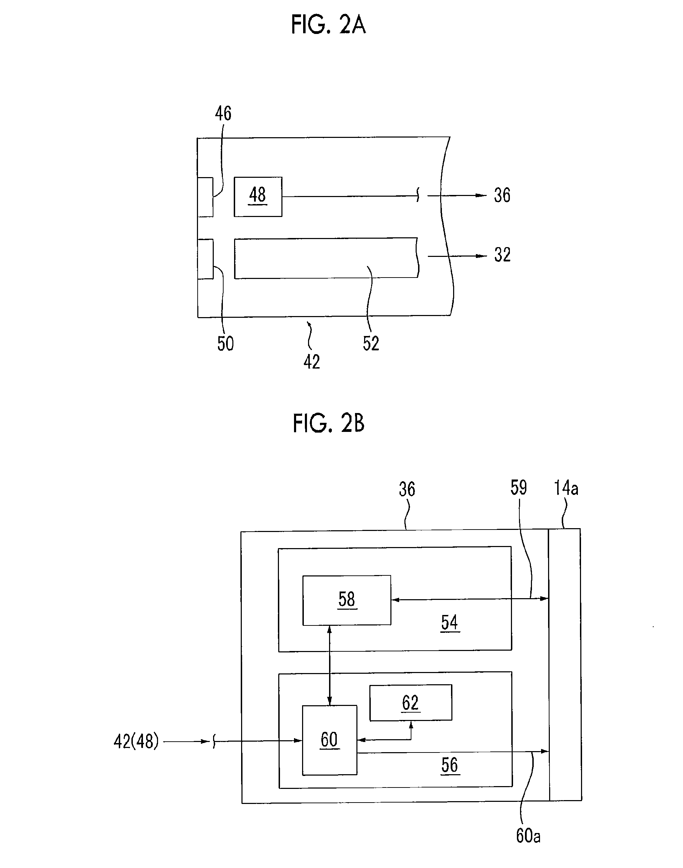

Fig. 2A is a block diagram schematically illustrating a configuration of the scope portion 42.

As shown in Fig. 2A, the scope portion 42 is provided with an imaging lens 46, a CCD sensor ((solid-state) imaging element) 48, an illumination lens 50, and an optical guide 52.

Furthermore, although not shown in the drawings, the scope portion 42 is provided with clamp channels and clamp holes through which various treatment tools such as a clamp are inserted and air/water supply channels and an air/water supply holes which are used to supply air and water therethrough. The clamp channels communicate with clamp insertion holes provided in the operation means 28 through the curved portion 40 and the flexible portion 38, and the air/water supply channels communicate with a suction means, an air supply means, and a water supply means of the connector 32 through the curved portion 40, the flexible portion 38, the operation means 28, and the universal cord 30.

The optical guide 52 is inserted up to the connector 32 connected to the light source device 16 through the curved portion 40, the flexible portion 38, the operation means 28, and the universal cord 30.

The illumination light emitted from the light source device 16 to be described later is incident from the connector 32 to the optical guide 52, is propagated through the optical guide 52, and is incident from the front end of the optical guide 52 to the illumination lens 50 in the scope portion 42, whereby the light is radiated from the illumination lens 50 to the observation portion.

Further, the image of the observation portion irradiated with the illumination light is formed on the light receiving surface of the CCD sensor 48 by the imaging lens 46.

The output signal of the CCD sensor 48 is sent through the signal line from the scope portion 42 to the video connector 36 (an AFE substrate 56 which will be described later) through the curved portion 40, the flexible portion 38, the operation means 28, the universal cord 30, and the connector 32.

In the case of an ordinary observation (diagnosis), the endoscope 12 is used in the state where the video connector 36 is connected to a connection portion 14a of the processor device 14 and the connector 32 is connected to a connection portion 16a of the light source device 16.

Furthermore, as in the ordinary endoscope, the connector 32 is connected with a suction means or an air supply means which suctions or supplies air to the observation portion, a water absorbing means which sprays water to the observation portion, and the like.

Fig. 2B is a block diagram schematically illustrating a configuration of the video connector 36.

A control substrate 54 and an AFE (Analog Front End) substrate 56 are disposed in the video connector 36 of the endoscope 12.

The control substrate 54 is used to control the endoscope 12, and a control means (CPU) 58 controlling the endoscope 12 is disposed thereon. Further, an image correction means 60 and a memory 62 are disposed on the AFE substrate 56.

The endoscopic device 10 of the present invention performs image correction concerned with variations in the characteristics of the CCD sensor 48, such as sensitivity unevenness correction or defective pixel correction in the endoscope 12.

The image correction means 60 performs such image correction using a correction parameter, and is desirably configured as a logic device (a so-called programmable logic device) such as an FPGA (Field Programmable Gate Array) which is able to rewrite a program therein.

The memory 62 stores a correction parameter so as to correct an image using the image correction means 60. The memory 62 may be configured as any one of various known memories (storage meanss) such as an EEPROM or a DRAD which is able to rewrite data therein. Further, the memory 62 may be of a volatile type or a non-volatile type. However, in the case of the nonvolatile memory, there is a need to generate and write a correction parameter to be described later whenever activating the endoscope.

The image correction performed by the image correction means 60 in the endoscope 12 is not particularly limited, and various image corrections (image processes) may be exemplified.

As an example, any one or more may be exemplified from sensitivity unevenness correction (sensitivity variation correction (gain unevenness correction)), offset correction, defective pixel correction, white balance adjustment, color chroma correction, gamma correction(grayscale correction), and the like. Especially, sensitivity unevenness correction and offset correction may be appropriately exemplified.

Each correction in the image correction means 60 may be performed by a known method in which image data is processed by using a correction parameter or the like generated in advance and stored in the memory 62. For example, in the case of sensitivity unevenness correction, image data of each pixel may be multiplied by a corresponding sensitivity unevenness correction parameter. Further, in the case of offset correction, a corresponding offset correction parameter may be subtracted from image data of each pixel. Furthermore, in the case of defective pixel correction, a defective pixel stored as a correction parameter may be compensated by using peripheral pixels.

Furthermore, if necessary, correction parameters respectively corresponding to a special light observation and a white light observation are stored in the memory 62 in accordance with the type of image correction to be performed, and the image correction means 60 may perform image correction using a correction parameter in accordance with the observation light.

The correction parameter stored in the memory 62 may be updated at arbitrary timing in necessary (the endoscope 12 may be corrected at arbitrary timing). The correction of the endoscope 12 may be performed by a known method.

Further, in order to generate a correction parameter described below and write the correction parameter to the memory 62, the correction parameter may be generated at the time of factory shipment by using a personal computer or a dedicated device and may be supplied and stored in a memory 58 of the endoscope 12.

Alternatively, the parameter may be updated at a predetermined interval, for example, when activating the endoscope, once a day, once a week, and the like.

Furthermore, although not shown in the drawings, a correlated double sampling circuit, an amplifier, an A/D converter, and the like may be disposed on the AFE substrate 56 in addition to the image correction means 60 and the memory 62.

The output signal of the CCD sensor 48 is first processed by the correlated double sampling so as to remove noise therefrom, is amplified by the amplifier, is converted into a digital signal by the A/D converter, and then is supplied to the image correction means 60.

In the endoscopic device 10 shown in the drawing, when the video connector 36 is connected to the connection portion 14a of the processor device 14, the control means 58 is connected to the processor device 14 through a serial I/F (interface) 59 such as RS232C.

Further, the image correction means 60 is connected with an image signal line (for example, a parallel bus) 60a which is used to output an image to an external device. When the video connector 36 is connected to the connection portion 14a of the processor device 14, the processor device 14 and the image correction means 60 are connected to each other through a parallel I/F.

Furthermore, the device shown in the drawing has a configuration in which the control substrate 54 and the AFE substrate 56 are provided in the video connector 36 of the endoscope 12, but the present invention is not limited thereto.

For example, if possible, at least one of the control substrate 54 and the AFE substrate 56 may be disposed in the scope portion 42. Further, at least one of the control substrate 54 and the AFE substrate 56 may be disposed in the connector 32 connected to the light source device 16. Further, at least one of the control substrate 54 and the AFE substrate 56 may be disposed in the operation means 28.

In the endoscopic device 10, in the case of an ordinary observation (diagnosis), as described above, the endoscope 12 is used in the state where the video connector 36 is connected to the connection portion 14a of the processor device 14 and the connector 32 is connected to the connection portion 16a of the light source device 16.

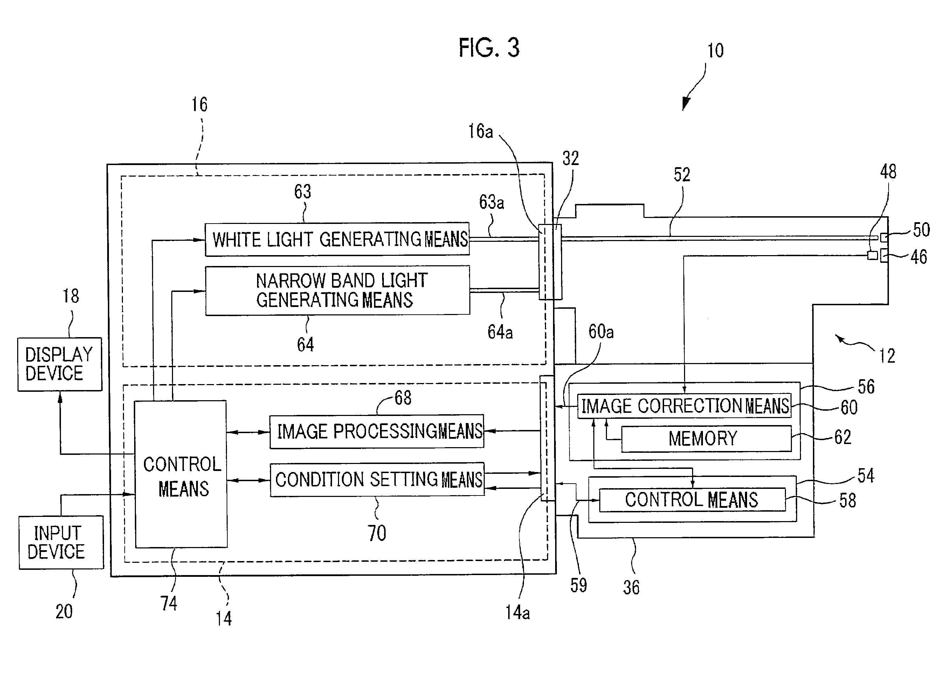

Fig. 3 is a block diagram schematically illustrating a configuration of the endoscopic device 10.

The light source device 16 is a known illumination device that radiates illumination light used for observation using the endoscope 12. As shown in Fig. 3, the light source device 16 of the example shown in the drawing includes a narrow band light generating means 64 which is used for narrow band light observation in addition to a white light generating means 63 which is used for ordinary observation.

Furthermore, in the present invention, the light source device 16 is not limited to the configuration. For example, the light source device 16 may include only the white light generating means 63 or include an observation light generating means which is used for special light observation other than narrow band light observation, such as an infrared light generating means generating infrared light instead of the narrow band light generating means 64 or together with the narrow band light generating means 64.

The white light generated by the white light generating means 63 is propagated to the connection portion 16a through an optical guide 63a, and the narrow band light generated by the narrow band light generating means 64 is propagated to the connection portion 16a through an optical guide 64b.

Since the connector 32 of the endoscope 12 is connected to the connection portion 16a, both observation lights are propagated from the connection portion 16a the optical guide 52 of the endoscope 12, and is propagated to the scope portion 42 through the optical guide 52, whereby the observation light is radiated from the illumination lens 50 to the observation portion.

The processor device 14 is used to perform a predetermined process on an image acquired by the endoscope 12 and display the image on the display device 18, and includes an image processing means 68, a condition setting means 70, and a control means 74.

The image (the image data) acquired by the CCD sensor 48 is corrected by the image correction means 60 of the video connector 36, is supplied to the processor device 14 by the image signal line 60a, is subjected to various image processes by the processor device 14 (the image processing means 68), and then is displayed on the display device 18.

Furthermore, the processor device 14 and the light source device 16 may, of course, include various parts provided in the processor device and the light source device of the known endoscopic device, such as a storage device or a power supply device, in addition to the parts shown in the drawings.

The control means 74 is a part that controls the processor device 14 and controls the overall part of the endoscopic device 10.

The image processing means 68 is used to perform various image processes such as a process in accordance with a command input by the input device 20 on an image acquired by the endoscope 12 and use the image as an image (image data) to be displayed on the display device 18.

Furthermore, the image process performed by the image processing means 68 is not particularly limited, and various known image processes such as noise removing, outline emphasizing (sharpening) may be used. Further, such image processes may be performed by the known method used in the endoscopic device.

The condition setting means 70 is used to generate a correction parameter (image correction condition) used in the image correction performed by the image correction means 60 of the video connector 36 or detect a defective pixel and set an image process condition or the like in the image processing means 68.

Furthermore, in the present invention, the setting of the image process condition in the image processing means 68, the generating of the correction parameter in the image correction means 60 of the endoscope 10, the detecting of the defective pixel, or the like may be performed by the known method in accordance with the process to be performed.

The correction parameter set by the condition setting means 70 and used in the image correction means 60 is sent to the image correction means 60 of the endoscope 12, and is written (updated) to the memory 62 by the image correction means 60.

Hereinafter, the endoscopic device 10 of the present invention will be more specifically described by referring to the generating of the correction parameter and the writing of the correction parameter to the memory 62.

When generating the correction parameter (correcting the endoscope), first, a correction image for generating the correction parameter is created.

When a command for generating the correction parameter (a command for correcting the endoscope 12) is input by the input device 20, a control means 74 displays a notice informing the acquisition for generating the correction image on the display device 18.

As an example, the correction image is created by photographing a subject such as a white subject with the same concentration using the endoscope 12.

In order to create the correction image, the image acquired by the endoscope 12 is supplied to the condition setting means 70 and the process described below is performed. Furthermore, at this time, the acquired image (the image data) is sent to the processor device 14 by the image signal line 60a without performing any process in the image correction means 60, and is supplied to the condition setting means 70.

Furthermore, in order to generate a correction parameter for offset correction before or after creating the correction image, the image of the observation portion may be acquired while the scope portion 42 is completely shielded from light and be supplied to the condition setting means 70 so as to generate an offset correction parameter (offset). The correction parameter may be generated by the known method.

Here, the correction image may be created from one image (one frame), but it is desirable to create the correction image in a manner such that a predetermined number of images (a predetermined number of frames) set in an appropriate way are obtained and are averaged by the condition setting means 72.

The condition setting means 72 acquired (created) the correction image creates the correction parameter used for the image correction in the image correction means 60 of the endoscope 12 by analyzing the correction image.

The method of generating the correction parameter is not particularly limited, and the correction image may be created by a known method in accordance with the image correction performed by the image correction means 60.

For example, in the case of a correction parameter for white balance adjustment, the correction parameter may be obtained by calculating a correction coefficient for each pixel, where the correction coefficient is used to correct an R-image and a B-image (to obtain a balance of an R-image and a B-image with respect to a G-image) to obtain a white image based on, for example, a G-image in the correction image.

In the case of a correction parameter for sensitivity unevenness correction, the average value of an arbitrary area (including all pixels) is calculated and is multiplied in the correction image. Then, a correction coefficient in which the pixel value is equal to the average value is calculated for each pixel, and is used as the correction parameter.

In the case of a correction parameter for defective pixel correction, the average value of the correction image is calculated, a pixel equal to or larger than a predetermined threshold value or different from the average value is detected as a defective pixel, and then the position of the detected defective pixel is used the correction parameter.

Further, in the case of a correction parameter for offset correction (dark state correction), the image acquired while the scope portion 42 is shield from light may be used as a correction parameter for offset correction.

When the condition setting means 70 generates a correction parameter, the correction parameter is supplied to the endoscope 12 and is written (stored) to the memory 62.

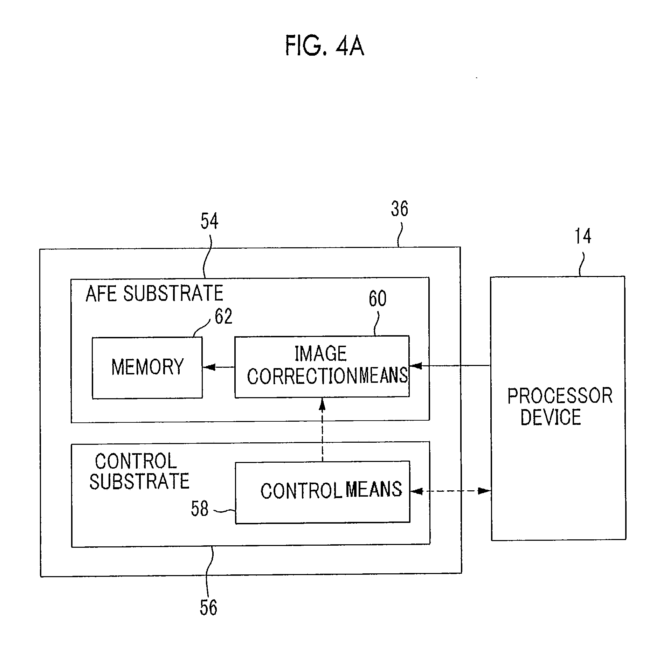

In the endoscopic device 10 of the example shown in the drawings, the writing of the correction parameter to the memory 62 is performed through the image correction means 60 in the manner conceptually shown in Fig. 4A. Furthermore, in Figs. 4A to 4D, the solid line indicates the flow of the correction parameter and the dashed line indicates the flow of the control signal.

When the condition setting means 70 creates a correction parameter, the processor device 14 (the control means 74) outputs a signal instructing the writing of the correction parameter to the control means 58 disposed in the video connector 36 (the control substrate 54) of the endoscope 12.

The control means 58 receiving the signal sends the correction parameter received from the processor device 14 to the image correction means 60. Although the image correction means 60 generally outputs an image to the external device from the image signal line 60a, the image correction means 60 receiving the command is made to enter a state where data may be received from the external device and the I/F of the image signal line 60a is made to enter a state where data may be transmitted from the external device.

When it becomes a state where the image correction means 60 may receive the correction parameter, the correction parameter is transmitted from the processor device 14 to the image correction means 60.

Further, the image correction means 60 writes the sent correction parameter to a predetermined area of the memory 62.

When the image correction means 60 writes all correction parameters to the memory 62 (the update of the correction parameter is ended), the writing (or the correcting) of the correction parameter in the endoscope 12 is ended.

Accordingly, the image correction means 60 of the endoscope 12 corrects the image acquired by the CCD sensor 48 by using the correction parameter written to the memory 62 later on.

As obvious from the description above, according to the endoscopic device 10 of the present invention, since the correction parameter may be easily written to the memory 62 included in the endoscope, the endoscope 12 may output an image subjected to image correction correcting variations in the characteristics of the CCD sensor 48, such as sensitivity unevenness correction or defective pixel correction, and may easily update the correction parameter for performing the image correction, that is, correct the endoscope if necessary.

Furthermore, the correction parameter may be stored or updated by using a connection originally provided in the endoscope without further providing a new device, a signal line, a connector, an I/F, or the like other than the image correction means 60 (FPGA or the like) or the memory 62 necessary for performing the image correction in the endoscope 12. For this reason, there is no concern of an increase in cost of the endoscope 12 or an increase in size of the part. Furthermore, there is no need to open the inside of the endoscope 12 in order to update the correction parameter.

Further, in the above-described example, since the correction parameter is written from the processor device 14 to the memory 62 using the image correction means 60 through the image signal line 60a as the parallel bus, the correction parameter may be rapidly written.

In the example shown in Figs. 3 and 4A, although the correction parameter is transmitted from the processor device 14 using the image signal line 60a through which the image corrected by the image correction means 60 is output to the outside, the present invention is not limited thereto.

That is, the present invention may use various configurations in which the existing signal line or the existing I/F are used as long as the correction parameter is written to the memory 62 through the image correction means 60.

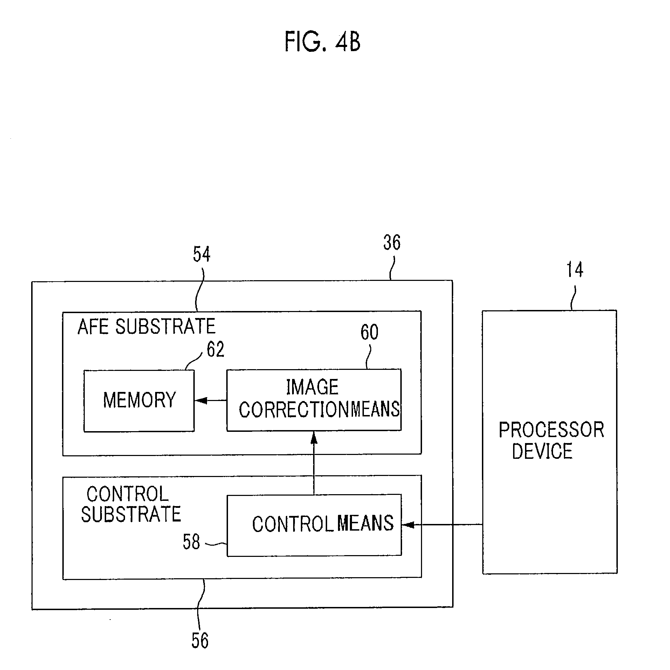

As an example, as shown in Fig. 4B, the correction parameter may be transmitted from the processor device 14 by using the serial I/F 59 used for the communication between the control means 58 and the processor device 14.

That is, when the condition setting means 70 of the processor device 14 creates the correction parameter in the same manner as above, the processor device 14 instructs the control means 58 to start the transmission of the correction parameter, and transmits the correction parameter to the control means 58 through the serial I/F 59.

Further, the control means 58 sends the received correction parameter to the image correction means 60. The image correction means 60 writes the sent correction parameter to a predetermined area of the memory 62 in the same manner as above.

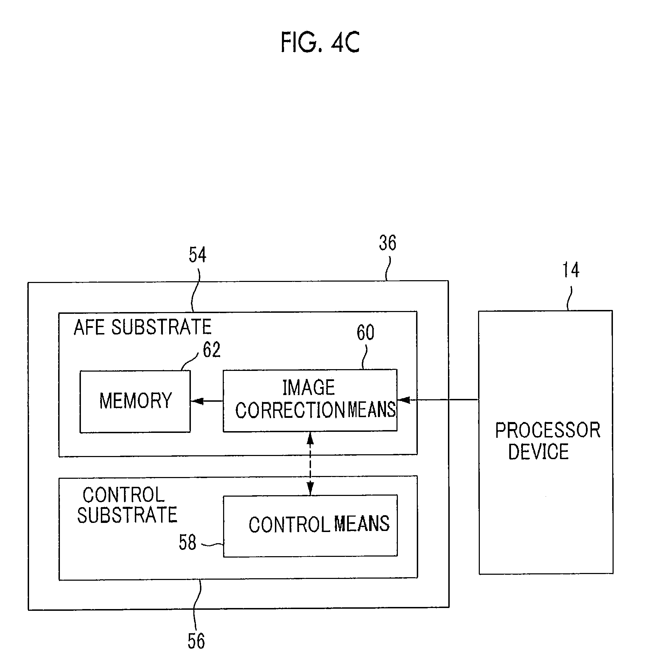

Alternatively, as shown in Fig. 4C, the correction parameter may be transmitted from the processor device 14 by connecting the serial I/F 59 to the image correction means 60 instead of the control means 58. At this time, the communication between the control means 58 and the processor device 14 is performed through the image correction means 60.

In this example, when the condition setting means 70 of the processor device 14 creates the correction parameter in the same manner as above, the processor device 14 generates a notice informing the transmission of the correction parameter in the control means 58 through the image correction means 60. Subsequently, the processor device 14 transmits the correction parameter to the image correction means 60 through the serial I/F 59.

The image correction means 60 writes the sent correction parameter to a predetermined area of the memory 62 in the same manner as above.

In the above-described example, the processor device 14 (the control device) generates the correction parameter, but the present invention is not limited thereto. For example, a configuration may be used in which the image correction means 60 generates the correction parameter and the image correction means 60 writes the correction parameter to the memory 62.

At this time, the processor device 14 performs only the display or the control of the display device 18.

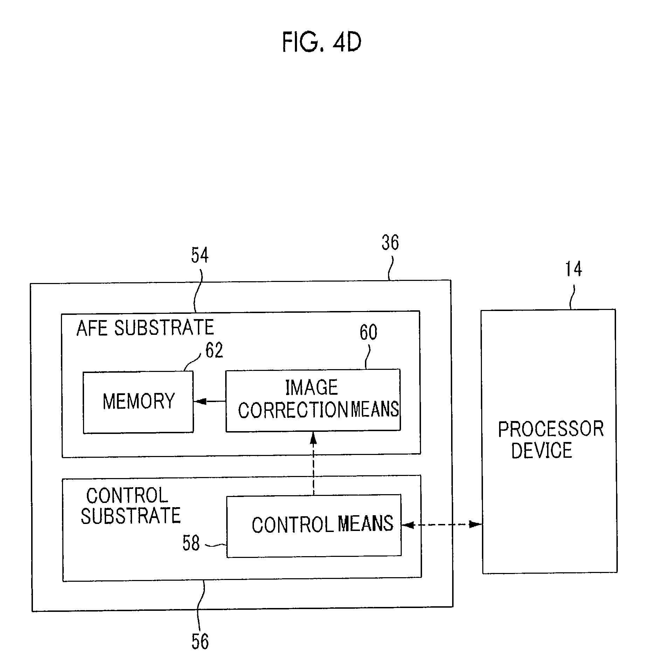

Fig. 4D illustrates an example thereof.

When a command for updating the correction parameter is generated by the input device 20 or the like, the processor device 14 generates a command for updating the correction parameter to the control means 58. Further, the control means 58 instructs the image correction means 60 to update the correction parameter.

Then, in the same manner as above, a photographing command for creating the correction image in the display device 18 is performed, and a photographing operation is performed so as to create the correction image.

The acquired image is sent to the image correction means 60. The image correction means 60 creates, for example, a correction image by averaging the acquired image in the same manner as above. Subsequently, the image correction means 60 generates a correction parameter by analyzing the created correction image in the same manner as above.

The image correction means 60 generating the correction parameter writes the generated correction parameter to a predetermined area of the memory 62 in the same manner as above.

In the above-described example, the processor device 14 processing an image acquired by the endoscope 12 is used as the control device of the present invention, but the present invention is not limited thereto.

For example, a computer (PC) different from the processor device 14 may be configured to be connected to the video connector 36 or the connector 32 of the endoscope 12, and the device may perform the same operation or process as that of the processor device 14 in the above-described respective examples. Further, a dedicated control device generating the correction parameter of the present invention and performing the same operation or process as that of the processor device 14 may be used.

While the endoscopic device of the present invention has been described, the present invention is not limited to the above-described embodiment, and various improvements or modifications may be, of course, made within a scope without departing from the concept of the present invention.

The present invention may be appropriately used in a medical treatment site using an endoscope.

## Claims

1. An endoscopic device comprising:
an endoscope that acquires an image using an imaging element;
a control device connected to the endoscope; and
a parameter generating means that generates a correction parameter for correcting the image acquired by the imaging element,
wherein the endoscope includes a correction means that corrects the image acquired by the imaging element using the correction parameter and a storage means that stores the correction parameter, and
wherein the parameter generating means generates the correction parameter in accordance with a command generated by the control device, and stores the generated correction parameter in the storage means using the correction means.

2. The endoscopic device according to claim 1,
wherein the control device includes the parameter generating means.

3. The endoscopic device according to claim 2,
wherein an image signal line used to output the image corrected by the correction means from the endoscope to an external device is connected to the control device, and
wherein the correction parameter is supplied from the control device to the correction means using the image signal line, and the correction means stores the correction parameter in the storage means.

4. The endoscopic device according to claim 1,
wherein the correction means includes the parameter generating means.

5. The endoscopic device according to any one of claims 1 to 4,
wherein the correction means is configured as a logic device which is able to rewrite a program therein.
